(19) Europäisches Patentamt
European Patent Office
Office européen des brevets

(11) Veröffentlichungsnummer: **0 110 316**
**A2**

# (12) EUROPÄISCHE PATENTANMELDUNG

(21) Anmeldenummer: **83111722.1**

(22) Anmeldetag: **23.11.83**

(51) Int. Cl.³: **C 07 D 213/06**

(30) Priorität: **27.11.82 DE 3244032**

(43) Veröffentlichungstag der Anmeldung: **13.06.84**
**Patentblatt 84/24**

(84) Benannte Vertragsstaaten: **BE CH DE GB IT LI NL**

(71) Anmelder: **HOECHST AKTIENGESELLSCHAFT,**
**Postfach 80 03 20, D-6230 Frankfurt am Main 80 (DE)**

(72) Erfinder: **Bicker, Richard, Dr., Brunnenstrasse 40,**
**D-6237 Liederbach (DE)**

## (54) Verfahren zur Herstellung von Pyridin.

(57) Pyridin wird hergestellt durch Ammoxidation (= Umsetzung mit Sauerstoff oder einem sauerstoffhaltigen Gas und Ammoniak, gegebenenfalls in Gegenwart von Wasserdampf) von Cyclopentadien in Gegenwart eines (Fällungs-) Kieselgels als Katalysator, welches insbesondere einen niederen $Al_2O_3$- und/oder $TiO_2$-Gehalt in oberflächennahen Bereichen sowie eine Feststoffsäurestärke entsprechend einem pka-Wert von $< +4,0$ besitzt. Der Kieselgel-Katalysator soll außerdem eine Oberfläche zwischen etwa 200 und 800 m²/g und ein Porenvolumen zwischen etwa 0,2 und 2,0 cm³/g aufweisen; die Reaktionstemperatur liegt zwischen etwa 200 und 400 °C.

Das Verfahren liefert durchweg Pyridin-Selektivitäten zwischen etwa 40 und 60% und keine vom Pyridin schwer abtrennbaren Pyridin-Homologen.

EP 0 110 316 A2

## Verfahren zur Herstellung von Pyridin

Pyridin ist ein wertvolles Lösemittel bei organischen Reaktionen, Reagenz zur Bindung freiwerdender Säuren, sowie Ausgangs- und Zwischenprodukt auf verschiedenen Sachgebieten, wie z.B. dem Pflenzenschutz- und Pharmasektor.

Die Gewinnung des Pyridins erfolgte früher praktisch ausschließlich aus dem Steinkohlenteer. In neuerer Zeit wurde diese Art der Pyridingewinnung durch synthetische Methoden verdrängt, die durchweg von einfachen aliphatischen Ausgangsprodukten - insbesondere von niederen aliphatischen Aldehyden oder Kohlenwasserstoffen - ausgehen. Technische Bedeutung hat hier vor allem die Pyridinsynthese aus Acetaldehyd und Ammoniak unter Zusatz von Formaldehyd an einem Aluminiumsilikat-Katalysator erlangt (vgl. Ullmann's Encyklopädie der technischen Chemie Bd. 19(1980) S. 593). Die Pyridinausbeute soll bei diesem Verfahren bis zu etwa 40 % d. Th. betragen. Der Rest besteht im wesentlichen aus höheren Pyridinhomologen (Picoline etc.; vgl. Abbildung 1 auf S. 593 der vorerwähnten Literaturstelle).

Der Anfall des Pyridins im Gemisch mit seinen höheren Homologen ist der früheren Pyridingewinnung aus dem Steinkohlenteer und den neueren - insbesondere den neueren technischen - Synthesemethoden gemeinsam. Daher heißt es auch auf S. 592 des vorgenannten Bandes von Ullmann's Enzyklopädie der technischen Chemie, daß Pyridin "sowohl aus den natürlichen Vorkommen, z.B. dem Steinkohlenteer, als auch bei den technisch ausgeübten Syntheseverfahren stets zusammen mit alkylierten Pyridinen (Pyridin-Basen) gewonnen wird".

Etwa zur Bindung freiwerdender Säuren oder auch als Lösemittel bei verschiedenen organischen Reaktionen können derartige Gemische des Pyridins mit seinen höheren Homologen

sicher häufig ohne weiteres eingesetzt werden. Für andere Zwecke - insbesondere wenn Pyridin Ausgangs- oder Zwischenprodukt für weitere Synthesen ist - ist jedoch meist reines Pyridin, frei von höheren Homologen, erforderlich. In diesen Fällen muß das Pyridin also aus seinem Gemisch mit höheren Homologen, wie es bei den üblichen Gewinnungsmethoden anfällt, getrennt werden, was wegen der sehr ähnlichen Eigenschaften (insbesondere Siedepunkte) einen nicht unerheblichen Aufwand erfordert.

Man hat daher auch bereits versucht, Pyridinsynthesen zu entwickeln, bei denen keine oder jedenfalls praktisch keine Pyridinhomologen mit entstehen.

Ein vorteilhaftes derartiges Verfahren besteht in der Ammoxidation von Cyclopentadien (= Reaktion von Cyclopentadien mit Ammoniak $NH_3$ und Sauerstoff oder einem molekularen Sauerstoff enthaltenden Gas, gegebenenfalls in Gegenwart von Wasserdampf)
unter Verwendung von Kieselgel, $\gamma$- $Al_2O_3$ und/oder Aluminiumsilikat(en) in tellurfreier Form, gegebenenfalls dotiert mit Oxiden von Übergangsmetallen und/oder von Gallium und/oder Indium,
mit einer Oberfläche zwischen etwa 75 und 800 $m^2/g$ und einem Porenvolumen zwischen etwa 0,2 und 2 $cm^3/g$ als Katalysator
bei Temperaturen von etwa 200 bis 400°C, vorzugsweise von etwa 250 bis 350°C (EP-OS 46897).

Bei dieser Methode entstehen keine vom Pyridin schwer abtrennbare Pyridin-Homologen. In dem besten der 12 Beispiele (Beispiel Nr. 12 - Katalysator: mit einer bestimmten Menge $V_2O_5$ dotiertes Kieselgel) ist eine Pyridin-Selektivität (= auf umgesetztes Cyclopentadien bezogene Pyridinausbeute) von 44,7 % angegeben (bei Umsatz etwa 83 %). Im sämtlichen übrigen Beispielen liegt die Pyridin-Selektivität jedoch

bei höchstens nur etwa 21 % (Umsatz meist zwischen etwa 50 und 100%). In dem einzigen dieser Beispiele, welches mit einem undotierten Katalysator arbeitet (Beispiel Nr. 11; Pyridinselektivität 20,8 % bei Umsatz 98,4 %) ist der Katalysator ein handelsübliches Kieselgel, nämlich Kieselgel E der Firma BASF AG, Ludwigshafen. Da dieses Produkt nach der Lieferspezifikation gemäß der BASF-Broschüre "Kieselgele für die Adsorption von Gasen und Flüssigkeiten" GCV/A - 4/78 noch Spuren $Na_2O$ und Sulfat (je 0,03 %) enthält, handelt es sich dabei wohl um ein auf an sich übliche Weise aus einer wässrigen Wasserglas-($Na_2SiO_4$)lösung und Schwefelsäure ($H_2SO_4$) erhaltenes (Fällungs-)Kieselgel. Unter den in diesem Kieselgel vorhandenen spurenweisen Verunreinigungen ist laut Lieferspezifikation auch 0,1 % $Al_2O_3$, was wohl vom Natriumsilikat herstammt und daher in dem Kieselgel gleichmäßig verteilt ist.

Da nach sämtlichen Beispielen der EP-OS Nr. 46897 - mit Ausnahme des aus der Reihe fallenden Beispiels Nr. 12 - Pyridinselektivitäten von höchsten nur etwa 21 % erreicht werden, was insbesondere für technische Belange noch verbesserungsbedürftig ist, bestand die Aufgabe, das ansonsten recht günstige Verfahren der EP-OS Nr. 46897 noch in Richtung auf höhere Pyridinselektivitäten zu verbessern.

Diese Aufgabe konnte erfindungsgemäß durch den Einsatz eines ganz speziellen Kieselgel-Katalysators gelöst werden, der sich insbesondere durch einen niederen $Al_2O_3$- und/oder $TiO_2$-Gehalt in oberflächennahen Bereichen auszeichnet.

Erfindungsgegenstand ist daher ein Verfahren zur Herstellung von Pyridin durch Ammoxidation von Cyclopentadien unter Verwendung eines (Fällungs-) Kieselgel-Katalysators mit einem Gehalt an $Al_2O_3$ und /oder einem Übergangsmetalloxid
einer Oberfläche zwischen etwa 200 und 800 $m^2/g$ und
einem Porenvolumen zwischen etwa 0,2 und 2,0 $cm^3/g$

bei einer Temperatur zwischen etwa 200 und 400°C, vorzugsweise zwischen etwa 250 und 350°C,
das dadurch gekennzeichnet ist, daß der (Fällungs-) Kieselgel-Katalysator einen niederen $Al_2O_3$- und/oder $TiO_2$-Gehalt in oberflächennahen Bereichen
sowie eine Feststoffsäurestärke entsprechend einem pka-Wert von kleiner als +4,0 besitzt.

Der $Al_2O_3$-Gehalt des Katalysators beträgt vorzugsweise etwa 0,01 bis 8 Gew.-%, insbesondere etwa 0,5 bis 2,5 Gew.-%, der $TiO_2$-Gehalt vorzugsweise etwa 0,1 bis 5 Gew.-%, insbesondere etwa 1 bis 2,5 Gew.-%, jeweils in den oberflächennahen Bereichen.

Durch den Einsatz dieser speziellen Katalysatoren gelingt es, die Pyridinselektivitäten auf durchweg zwischen etwa 40 und 60 % bei Umsätzen zwischen etwa 50 und 100 %, meist sogar zwischen etwa 90 und 100 %, zu erhöhen, ohne daß dabei etwa auch merkliche Mengen an Pyridinhomologen entstehen. Dieses Ergebnis war außerordentlich überraschend, weil nicht zu erwarten war, daß durch eine derart einfache Katalysatorvariation eine solch erhebliche Verbesserung des Verfahrens der EP-OS Nr. 46897 möglich ist.

Die hier benutzten Katalysatoren können auf an sich bekannte Weise hergestellt werden und sind auch im Handel erhältliche Produkte.

Die Herstellung der Fällungs-Kieselgele geht üblicherweise aus von einer wässrigen Natriumsilikat-("Wasserglas"-) Lösung und verdünnter Schwefelsäure. Dabei werden dann - entsprechend etwa der Darstellung in Ullmann's Enzyklopädie der technischen Chemie Bd. 21 (1982), S. 460 - folgende Stufen durchlaufen:

wässr. $Na_2SiO_4$-lösung $\xrightarrow{\text{verd.} H_2SO_4}$ Kieselsol → Hydrogel → Xerogel
"Wasserglas"

Beim Zusammengeben von Wasserglas und Schwefelsäure bilden sich primär niedrigmolekulare Kieselsäuren. In Abhängigkeit vom pH-Wert kondensieren die Si-OH-Gruppen unter Wasserabspaltung weiter unter Bildung von Polykieselsäuren. Die dabei entstehenden Partikel mit einem Durchmesser von ca. 10 nm liegen in kolloidaler Lösung vor. Damit ist die erste Stufe der Kieselgel-Synthese - die Kieselsolbildung - abgeschlossen.

Aus dem Kieselsäuresol entsteht durch weitere Kondensation von oberflächenständigen Silanolgruppen ein dreidimensionales Netzwerk, das Kieselsäure-Hydrogel. Im Hydrogel findet auch nach vollzogener Gelierung eine weitere Kondensation von Si-OH-Gruppen statt.

Das Hydrogel wird gewaschen, um das $Na_2SO_4$ zu entfernen, und anschließend zu porösem Xerogel getrocknet. Durch unterschiedliche Nachbehandlung und Trocknung sind in bekannter Weise die gewünschten Oberflächen und Porenvolumina einstellbar.

Die für das erfindungsgemäße Verfahren erforderlichen (Fällungs-)Kieselgel-Katalysatoren mit einem niederen $Al_2O_3$- und/oder $TiO_2$-Gehalt in oberflächennahen Bereichen werden dadurch erhalten, daß man am Ende der Hydrogelbildung (vor der Strukturentwicklung oder noch besser nach abgeschlossener Strukturentwicklung des Hydrogels) mit einer wässrigen Al- und/oder Ti-Salzlösung versetzt; zweckmäßig sind wässrige $Al_2(SO_4)_3$ und $TiO(SO_4)$-Lösungen. Als Orientierungshilfe für die Menge der zu verwendenden Al- bzw. Ti-Salze kann folgende Beziehung dienen:

Einem $Al_2O_3$-Gehalt von 0,01 bis 8 Gew.-% in dem fertigen Kieselgel entspricht ein Atomverhältnis Si:Al = etwa 8500-10:1, und

einem $TiO_2$-Gehalt von 0,01 bis 5 Gew.-% in dem fertigen Kieselgel entspricht ein Atomverhältnis Si:Ti von etwa 13.300-25:1.

Die Weiterbehandlung (Auswaschen, Trocknung) geschieht dann wie üblich. Dabei werden aus den Al- und Ti-Salzen die Oxide gebildet. Da die Al- bzw. Ti-Salzbehandlung erst in einer späten Stufe der Kieselgel-Herstellung erfolgt, liegen das $Al_2O_3$ bzw. $TiO_2$ dann praktisch nur in den oberflächennahen Bereichen vor.

Derartige Kieselgele mit einem niedrigen $Al_2O_3$- oder $TiO_2$-Gehalt in oberflächennahen Bereichen (Cogele) werden z.B. von Firma Grace GmbH, Worms, angeboten.

Die für das erfindungsgemäße Verfahren eingesetzten Katalysatoren können aber z.B. auch durch eine Oberflächenbehandlung von bereits fertigem (Fällungs-)Kieselgel (Xerogel) etwa mit einer wässrigen $Al_2(SO_4)_3$-Lösung oder etwa mit einer alkoholischen $TiCl_4$-Lösung entsprechend der Vorschrift für die Herstellung von "aluminum- on-silica" in dem Artikel von K.H. Bourne, F.R. Cunnings und R.C. Pitkethly in J. Physical Chemistry, Vol. 74 No. 10, S. 2197 bis 2205, speziell S. 2198 linke Spalte, erhalten werden. Nach dieser Vorschrift sind direkt nur die entsprechenden Al-modifizierten Kieselgele zugänglich; für die Herstellung der Ti-modifizierten Gele ist anstelle der wässrigen Al-Salzlösung etwa eine alkoholische $TiCl_4$-Lösung zu verwenden.

Die Oberflächenbestimmung der Kieselgele erfolgt nach der bekannten Methode von S. Brunauer, P.H. Emmett und E. Teller, beschrieben z.B. in J. Am. Chem. Soc. 60 (2), S. 309 (1938).

Das Porenvolumen wird z.B. nach der Titrationsmethode von W.B. Innes, Analytical Chemistry 28 (3), S. 332 (1956), bestimmt.

Die Angabe der Feststoffsäurestärke entsprechend einem pka-Wert von kleiner als +4,0 bezieht sich auf die nach der Indikatormethode von C. Walling, J. Am. Chem. Soc. 72, S. 1164 (1950), und H.A. Benesi, J. Am. Chem. Soc. 78, S. 5490

(1956) erhaltenen Werte.

Ansonsten wird das erfindungsgemäße Verfahren genauso durchgeführt wie das Verfahren der EP-OS Nr. 46897; dies bedeutet die Durchführung unter den nachstehend detailliert beschriebenen Bedingungen. Als Oxidationsmittel für die erfindungsgemäße Ammoxidation kann sowohl reiner Sauerstoff als auch ein sauerstoffhaltiges Gas eingesetzt werden. Bevorzugt ist die Verwendung von Luft. Das Molverhältnis zwischen Sauerstoff und Cyclopentadien in dem dem Reaktionsraum zugeführten Gasgemisch liegt zweckmäßig im Bereich zwischen etwa 0,5 bis 75 zu 1, wobei ein Verhältnis im Bereich von etwa 1 bis 20:1 bevorzugt ist.

Auch das Molverhältnis zwischen Ammoniak und Olefin in dem Eingangsgasgemisch kann in weiten Grenzen schwanken. Zweckmäßig liegt das Molverhältnis bei etwa 0,5 bis 50 zu 1, vorzugsweise bei etwa 10 bis 30:1.

Wasserdampf ist als Verdünnungsmittel bei dem erfindungsgemäßen Verfahren nicht unbedingt notwendig; seine Anwesenheit ist jedoch zweckmäßig. Wenn in Gegenwart von Wasserdampf gearbeitet wird, soll das molare Verhältnis von Wasserdampf zu Cyclopentadien im Ausgangsgasgemisch etwa im Bereich von 10 bis 100:1, vorzugsweise von etwa 15 bis 65:1 liegen.

Die Reaktionstemperatur liegt im gleichen Bereich wie auch die Reaktionstemperatur des Verfahrens der EP-OS Nr. 46897, nämlich zwischen etwa 200 und 400°C, vorzugsweise zwischen etwa 250 und 350°C.

Wenn sich im Laufe der Reaktion auf dem Kieselgel Polymerabscheidungen bilden, kann eine Regenierung bzw. Reaktivierung des Katalysators durch eine Behandlung mit Luft bei etwa 300 bis 500°C erfolgen. Die Polymerabscheidungen werden dabei abgebrannt.

Die Ammoxidationsreaktion wird vorzugsweise bei etwa atmosphärischem oder geringfügig höherem Druck durchgeführt.

Die scheinbare Kontaktzeit ist nicht kritisch. Es können scheinbare Kontaktzeiten im Bereich von etwa 0,1 bis etwa 10 Sekunden angewandt werden. Im allgemeinen ist jedoch eine Kontaktzeit im Bereich von etwa 0,1 bis etwa 5 Sekunden bevorzugt.

Zur Durchführung des erfindungsgemäßen Verfahrens kann jede Apparatur eingesetzt werden, die zur Durchführung von Oxidationsreaktionen in der Dampfphase zum Einsatz kommen. DasVerfahren kann entweder kontinuierlich oder diskontinuierlich durchgeführt werden, wobei im Festbett oder in einer Wirbelschicht gearbeitet werden kann.

Es empfiehlt sich, das Ausgangsgasgemisch (Cyclopentadien, Ammoniak, Sauerstoff bzw. ein molekularen Sauerstoff enthaltendes Gas, sowie gegebenenfalls Wasserdampf) vorzuheizen. Das Katalysatorbett kann ein Festbett unter Anwendung grober Katalysatorteilchen sein, wie unregelmäßige Formkörper, Strangpreßlinge, Kugeln, Pellets oder Tabletten; es kann jedoch auch mit einem in Wirbelschicht angeordneten Katalysator durchgeführt werden.

Der den Reaktor verlassende Gasstrom enthält im wesentlichen Pyridin, Kohlenoxide, Acetonitril und Blausäure. Diese Umsetzungsprodukte können aus dem Gasstrom nach an sich bekannten Verfahren abgetrennt werden.

Eines dieser Verfahren besteht z.B. darin, das den Reaktor verlassende Gas mit kaltem Wasser zu waschen, wobei das Pyridin und das Acetonitril im wesentlichen aus dem Gasstrom entfernt werden. Zur besseren Absorption des Pyridins kann das Wasser auch angesäuert sein, wobei in diesem Fall auch gleichzeitig das nicht-umgesetzte Ammoniak neutralisiert

und Ammoniumcarbonatbildung aus nicht-umgesetztem Ammoniak und Kohlendioxid vermieden wird.

Die gebildete Blausäure verbleibt ebenfalls im Waschwasser. Der die Wasserwäsche verlassende Gasstrom enthält im wesentlichen nicht umgesetztes Olefin, Kohlendioxid, Stickstoff und Sauerstoff und kann bei geringerem Umsatz nach Abtrennung des Kohlendioxids dem Reaktor wieder zugeführt werden bzw. wird bei weitgehend vollständigem Umsatz verworfen.

Die engültige Gewinnung des Pyridins geschieht nach allgemein üblichen Methoden.

Das nicht-umgesetzte Cyclopentadien kann auch aus dem die Wasserwäsche verlassenden Gasstrom durch Extraktion mit einem unpolaren Lösungsmittel wie z.B. Methylenchlorid, entfernt und so zurückgewonnen werden.

Insbesondere wegen der erheblichen Erhöhung der Pyridinselektivität gegenüber dem Verfahren der EP-OS Nr. 46897 stellt die Erfindung einen beachtlichen Fortschritt auf diesem Gebiet dar.

Eine detaillierte Beschreibung des erfindungsgemäßen Verfahrens erfolgt in Verbindung mit den Ausführungsbeispielen. Die in den Beispielen angegebenen Ausbeuten, Umsätze und Selektivitäten sind wie folgt definiert:

$$\text{Ausbeute:} \quad \frac{\text{Menge gebildetes Pyridin}}{\text{Menge max. erwartetes Pyridin}} \cdot 100$$

$$\text{Umsatz:} \quad \frac{\text{Menge einges. Cyclopentadien} - \text{Menge zurückgew. Cyclopentadien}}{\text{Menge einges. Cyclopentadien}} \cdot 100$$

$$\text{Selektivität:} \quad \frac{\text{Ausbeute}}{\text{Umsatz}} \cdot 100$$

Der in den Beispielen eingesetzte Reaktor war ein Standardreaktor aus VA-Stahl mit einem Durchmesser von 2 cm. Der Reaktor besaß ein inneres axiales Rohr als Temperaturmeßseele, bei dem die Temperatur in der Katalysatorschüttung an mehreren Punkten mit einem Thermoelement gemessen werden konnte. Beheizt wurde das Reaktorrohr durch eine Salzschmelze. Die Gase wurden mittels Rotametern in den Reaktor eingemessen, das Cyclopentadien wurde aus einer gekühlten Waschflasche durch einen Stickstoffstrom in den Reaktor gefördert.

Sowohl das in den Reaktor eintretende Gasgemisch als auch das den Reaktor verlassende Gasgemisch wurden on-line von einem Gaschromatographen analysiert. Pyridinhomologe wurden dabei nicht gefunden.
Die eingesetzten Kieselgele hatten eine Korngrößenverteilung von etwa 1 bis 3 mm. Die angegebenen Werte für Umsätze, Ausbeuten und Selektivitäten stellen gaschromatographisch ermittelte Maximalwerte über einen Zeitraum von etwa 1 Stunde dar.

Die Beispiele wurden derart durchgeführt, daß 25 g des in der Tabelle näher spezifizierten Kieselgels in den VA-Stahlreaktor (Durchmesser 2 cm) eingefüllt wurden (resultierende Füllhöhe: 40 cm) und daß dann durch diesen Reaktor bei 300°C 0,4 g/h Cyclopentadien, 5 l/h NH, 17,5 l/Luft und 23 l/h Stickstoff - in Beispiel 2 zusätzlich noch 1 g/h Wasserdampf - während einer Versuchszeit von 4 bis 5 Stunden geleitet wurden. Das Resultat ist aus den letzten drei Spalten der Tabelle ersichtlich.

Die Katalysatoren für die Beispiele Nr. 1 bis 4 sind (Fällungs-)Kieselgele der Firma Grace GmbH, Worms, mit geringen Mengen $Al_2O_3$ bzw. $TiO_2$ in oberflächennahen Bereichen, hergestellt durch Imprägnierung des gefällten Gels mit einer Al- bzw. Ti-Salzlösung in einem späten Stadium der Hydrogelbildung, in den vorliegenden Fällen speziell nach abgeschlossener Strukturentwicklung des Hydrogels.

Bei dem in den Beispielen 1 und 2 eingesetzten Katalysator handelt es sich um das Al-Cogel Nr. 8 gemäß dem Blatt "Modifizierte Silica-Gele-Cogele" der Broschüre "Grace-Silica Gel" Dez. 1981/0-2-05.1 Gö.D der Firma Grace GmbH, bei dem Katalysator in Beispiel 3 um das Al-Cogel Nr. 10 und bei dem Katalysator in Beispiel 4 um das Ti-Cogel Nr. 2 gemäß dem gleichen Blatt der genannten Grace-Broschüre.

Der Katalysator für Beispiel 5 wurde hergestellt entsprechend der Vorschrift in dem Artikel von K.H. Bourne et al. in J. Physical Chemistry Vol. 74 No. 10 (1970), S. 2198 linke Spalte. Dazu wurde zuerst das 0,1 % $Al_2O_3$ enthaltende Kieselgel E der Firma BASF AG 2 Stunden bei 100°C mit 5 %iger Salpetersäure behandelt und dadurch praktisch Al-frei gewaschen. Dieses Kieselgel wurde dann mit einer Lösung von $Al_2(SO_4)_3 \cdot 18\ H_2O$ (0,1 molar) 48 Stunden bei 20°C überschichtet. Das Gel wurde dann gründlich sulfatfrei gewaschen und bei 110°C 16 Stunden getrocknet.

In Anlehnung an diese Vorschrift wurde auch der Katalysator für Beispiel Nr. 6 hergestellt. Dazu wurden 98 g desAl-frei gewaschenen Kieselgels E der Firma BASF AG mit einer Lösung von 2,37 g $TiCl_4$ in absolutem Ethanol imprägniert. Das imprägnierte Kieselgel wurde in eine Säule gegeben und mit Stickstoff das Ethanol vertrieben. Anschließend wurden die verbliebenen Ti-Cl-Bindungen durch Hindurchleiten von feuchter Luft hydrolysiert und dann wurde 6 Stunden bei 400°C im Luftstrom calciniert.

Tabelle

| Bsp. Nr. | Katalysator (Fällungs-)Kieselgel mit: $Al_2O_3$ [Gew.-%] in oberflächennahen Bereichen | $TiO_2$ [Gew.-%] | BET-Oberfläche [$m^2$/g] | Porenvolumen [$cm^3$/g] | pKa | Wasserdampf-Zusatz [g/h] | Umsatz [%] | Pyridin-Selektivität [%] | Ausbeute [%] |
|---|---|---|---|---|---|---|---|---|---|
| 1 | 0,9 | - | 526 | 1,41 | $< +3,3$ | - | 96,3 | 51,4 | 49,6 |
| 2 | 0,9 | - | 526 | 1,41 | $< +3,3$ | 1 | 98,7 | 60,5 | 59,7 |
| 3 | 1,2 | - | 406 | 1,61 | $< +3,3$ | - | 92,6 | 49,5 | 45,5 |
| 4 | - | 2,3 | 342 | 1,21 | $< +3,3$ | - | 98,5 | 49,1 | 48,4 |
| 5 | 1,3 | - | 650 | 0,27 | $< +3,3$ | - | 90,8 | 43,6 | 39,6 |
| 6 | - | 1 | 650 | 0,27 | $< +3,3$ | - | 96,7 | 42,6 | 41,2 |

<u>Patentansprüche:</u> HOE 82/F 239

1. Verfahren zur Herstellung von Pyridin durch Ammoxidation von Cyclopentadien unter Verwendung eines (Fällungs-) Kieselgel-Katalysators mit einem Gehalt an $Al_2O_3$ und/oder einem Übergangsmetalloxid einer Oberfläche zwischen etwa 200 und 800 $m^2/g$ und einem Porenvolumen zwischen etwa 0,2 und 2,0 $cm^3/g$ bei einer Temperatur zwischen etwa 200 und 400°C, vorzugsweise zwischen etwa 250 und 350°C, dadurch gekennzeichnet, daß der (Fällungs-)Kieselgel-Katalysator einen niederen $Al_2O_3$- und/oder$TiO_2$-Gehalt in oberflächennahen Bereichen sowie eine Feststoffsäurestärke entsprechend einem pka-Wert von $<$+4 besitzt.

2. Verfahren nach Anspruch 1, dadurch gekennzeichnet, daß der $Al_2O_3$-Gehalt des Katalysators etwa o,01 bis 8 Gew.-%, insbesondere etwa 0,5 bis 2,5 Gew.-%, beträgt.

3. Verfahren nach Anspruch 1, dadurch gekennzeichnet, daß der $TiO_2$-Gehalt des Katalysators etwa C,1 bis 5 Gew.-%, insbesondere etwa 1 bis 2,5 Gew.-%, beträgt.